(19) Europäisches Patentamt
European Patent Office
Office européen des brevets

(11) **EP 1 656 545 B1**

(12) **EUROPEAN PATENT SPECIFICATION**

(45) Date of publication and mention
of the grant of the patent:
**08.02.2012 Bulletin 2012/06**

(51) Int Cl.:
*G01N 1/30* (2006.01)   *G01N 15/14* (2006.01)
*G06K 9/00* (2006.01)   *G01N 21/64* (2006.01)

(21) Application number: **04816174.9**

(22) Date of filing: **10.08.2004**

(86) International application number:
**PCT/US2004/025981**

(87) International publication number:
**WO 2005/027037 (24.03.2005 Gazette 2005/12)**

(54) **DEVICES AND METHODS TO IMAGE OBJECTS BY TIME DELAY INTEGRATION**

VORRICHTUNGEN UND VERFAHREN ZUR ABBILDUNG VON OBJEKTEN MITTELS "TIME DELAY INTEGRATION"

DISPOSITIFS ET PROCEDES PERMETTANT D'IMAGER DES OBJETS PAR "TIME DELAY INTEGRATION"

(84) Designated Contracting States:
**AT BE BG CH CY CZ DE DK EE ES FI FR GB GR HU IE IT LI LU MC NL PL PT RO SE SI SK TR**

(30) Priority: **11.08.2003   US 494101 P**

(43) Date of publication of application:
**17.05.2006   Bulletin 2006/20**

(73) Proprietor: **IMMUNIVEST CORPORATION
Wilmington, DE 19899 (US)**

(72) Inventors:
• **GREVE, Jan,
Fac. TNW, University of Twente
7500 AE Enschede (NL)**

• **SCHREUDER, Frederick,
Biophysical Techniques Group
7500 AE Enschede (NL)**
• **TERSTAPPEN, Leon, W.W.M.
Huntingdon Valley, PA 19006 (US)**
• **TIBBE, Arjan, G.J.
NL-7424BL Deventer (NL)**

(74) Representative: **Hanson, William Bennett et al
Bromhead Johnson
19 Buckingham Street
London
WC2 6EF (GB)**

(56) References cited:
**WO-A1-02/054339      WO-A1-03/014400
US-A- 6 151 405      US-A1- 2002 012 910
US-B1- 6 365 362**

## Description

## Technical Field

This invention relates generally to devices and methods to obtain to scan and obtain images of objects, and more particularly to images reconstructed from partial sub-images of object such as cells obtained from biological fluids that are distributed in a two-dimensional plane. The scanning and imaging technique provided by the invention is especially advantageous for the imaging of cells that are aligned by magnetic means and examined by digitized optoelectronic means through Time-Delay-Integration.

## Background of the Invention

The identification and enumeration of circulating carcinoma cells of epithelial origin in the blood of cancer patients that may be present at frequencies of less than one carcinoma cell per ml of blood has been detected using magnetic enrichment and image cytometry as in, for example, CellTracks® Systems (Immunicon Corporation, Huntingdon Valley, PA). Using a combination of epithelial cell enrichment by magnetic means in combination with analysis by multi-parametric flow cytometry, significant differences in the number of "circulating tumor cells" were found between healthy individuals and patients with breast cancer (Racila et al., Proc. Nat. Acad. Sci. 95, 4589-4594, 1998). In several studies, such "circulating tumor cells" (CTC) were defined as events expressing the following characteristics: positive for the epithelial cell marker cytokeratin, negative for the leukocyte marker CD45, positive staining with a nucleic acid dye, and light scattering properties that are compatible with cells. However, morphometric confirmations of the detected events as cells and further molecular evidence is lacking in flow cytometric methods, but is clearly needed to assure that the detected rare events are indeed tumor cells derived from a primary tumor. Automated image analysis systems have been introduced to reduce subjective errors in cell classification between different operators in manual methods, but such prior art systems without preliminary cell enrichment steps still inherently lack sensitivity. Several automated cell imaging systems have been described or are commercially available for cell analysis. The system developed by Chromavision, ACIS™ or Automated Cellular Imaging System (Douglass et al., US patent 6,151,405) uses colorimetric pattern recognition by microscopic examination of prepared cells by size, shape, hue and staining intensity as observed by an automated computer controlled microscope and/or by visual examination by a health care professional. The system uses examination of cells on microscope slides and was designed for tissue sections. The SlideScan™ or MDS™ systems of Applied Imaging Corp. (Saunders et al., US patent 5,432,054) is described as an automated, intelligent microscope and imaging system that detects cells or "objects" by color, intensity, size, pattern and shape followed by visual identification and classification. In contrast to the ACIS system this system has the ability to detect fluorescent labels which provides more capability. However, these and other currently available methodologies are not sufficiently sensitive for accurate classification and typing of rare events such as circulating tumor cells in blood.

Thus, an imaging system (US 10/612,144) was developed to detect fluorescent signals at a relatively high speed, performing similar to standard flowcytometry. The system is based on the separation of cells by first labeling with immunomagnetic particles. These particles are colloidal paramagnetic paricles linked to a monoclonal antibody specific for a characteristic cell surface antigen found on the target cell. When sufficiently bound to the target population of cells, a magnetic field forces the immunomagnetic particle-cell complex to rise to the top of the chamber where the cells align between nickel lines. When targeting epithelial-derived cells with a monoclonal antibody to the Epithelial Cell Adhesion molecule (EpCAM), erythrocytes and other non-labeled cells separate from the labeled cells which rise up to the upper glass surface of the chamber. Since this is in a known volume, enumeration of cells can easily be determined. A red diode laser (635 nm) is focused as an elliptical beam on the chamber, inconjunction with the nickel lines. The reflection from the nickel lines is used as a signal for the tracking and focusing of the laser beam on the aligned cells. The sample chamber is moved with the X-Y stage in the Y direction and the laser is scanning one line at a time. The aligned cells are illuminated one by one and the emitted fluorescence is measured with photomultipliers. The advantages of this design are that only the target cells align on the inner surface of the viewing portion of the chamber and the aligned cells are easily relocated after the first analysis has been completed. Both benefits ensure that extremely small populations of target cells can be measured with the imaging system described in US 10/612,144 after magnetic enrichment. Scatter plots from fluorescent data, generated from multi-labeling experiments, can be used to select rare events. With the cells aligned along the inner surface of the viewing portion of the chamber, fluid underneath the cells can be replaced, and the cells can be further analyzed.

Prior technology has been based upon a standard full frame charge coupled device (CCD) camera with, for example, a frame rate of 25 frames per second. The cell is scanned with a moving stage or a scanning mirror. The images acquired with the camera are added together, forming a complete image of the cell. The disadvantage is that when scanning, the laser is only illuminating a small area of the cell. Therefore it is necessary to scan the object (or cell) to image the entire cell. To acquire enough photons and to prevent bluring due to the movement of the cell it is necessaayr to scan slowly. Another disadvantage of this type of imaging is that a full frame camera is not collecting photons during readout of the CCD. Consequently, all fluorescent photons emitted at that moment are useless. The camera is closed for readout for about 16% of the total time for a pixel area of 512x512 pixels at 25 fps and a fast readout rate of 40 Mhz.

Initially used in military airborne reconnaissance and satellite imaging, TDI technoogy allows for the capture of high quality images of moving objects. TDI imagings devices have also been applied in document scanning and industrial product inspection.

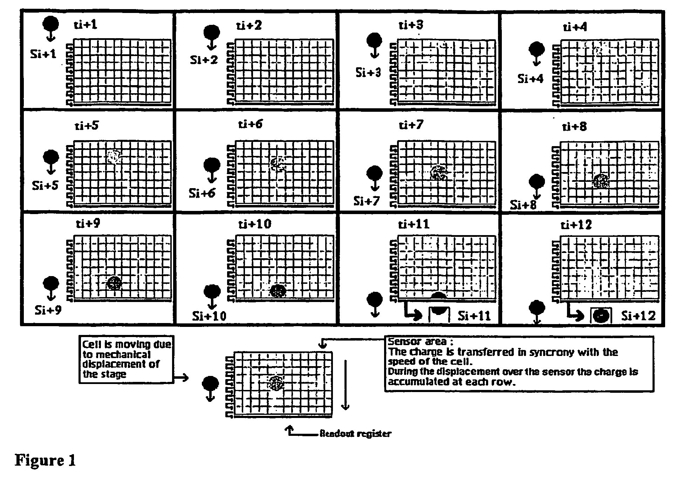

A TDI imager is a standard full frame CCD imager with a length (columns) which is much larger than its height (rows or TDI stages). This line scanner has 96 TDI rows which results in an enhanced sensitivity that is 96 times better than a standard line scanner with a single row of pixels ant the same readout speed. Figure 1 illustrates how a moving object is interpreted with TDI technology. $S_i$ represents the position of a moving object at time $t_i$. At time $t_i+3$, the first row of TDI CCD collects the light emitted by the moving object. The TDI shifts the collected charge from the first row to the second row with the same speed as the object that is moving from $S_i+3$ to $S_i+4$. Thus after each shift, the accumulated charges are added to photo-generated charges at the new site, integrating the charges along the TDI column. This process is repeated until the end of the sensor is reached. For example, the Dalsa Eclipse line scanner has the number of TDI stages at 96 which means the process is repeated 96 times.

TDI imaging has the advantage of enhanced photosensitivity and good signal-to-noise woithout affecting the output data rate. Consequently, high scan speeds and the ability to image at low light levels are obtained. The uniformity of pixel sensitivity is improved with the averaging over all the TDI stages, resulting in a more linear output. The dark current uniformity of the pixels is improved as a consequence of the averaging over all the TDI stages. Exposure is easily controlled through the scan speed of the object. Further, large objects are able to be imaged with a small CCD camera as they are moving.

Accordingly, the present invention seeks to improve upon traditional CCD technology as related to image cytometric analysis, such as CellSpotter™ Systems, and to permit detection, enumeration and accurate classification of rare target species, such as CTC in blood or other fluids.

WO 02/054339 A1 describes automated image analysis enabling identification of rare target cells aligned on surfaces, using automated laser scanning to generate sequential digitized x-y sub images that are combined to form reconstructed full images.

WO 03/014400 A1 describes the scanning of a biological sample with TDI by a CCD camera having columns and rows.

US 2002/0012910 A1 describes automatic multiplex sequencing of DNA with an integrated imaging hybridization chamber system in which a CCD camera is operated in TDI mode.

## Summary of the Invention

This invention improves upon the devices and methods that permit the application of novel imaging capabilities to such systems as the CellTracks® analysis system as described by Tibbe et al. (Nature Biotech. 17, 1210-13, 1999). The devices and methods described by Tibbe and in the disclosure of this invention can also be applied to other target objects. However, the primary application is rapid immunomagnetic selection of rare cells from blood followed by automated alignment of the isolated cells and automated image analysis. Briefly, in a preferred embodiment of the invention, after magnetic collection and enrichment from blood, the magnetically labeled cells are aligned along ferromagnetic lines of nickel (Ni) and are scanned by a laser focused by means of a conventional objective lens such as from a compact disk player. Since the cells have been selectively stained with one or more fluorescent labels, the measured fluorescence emissions and the intensities can be used to identify or classify the cell type.

No liquid flow system is required by the system of the present invention. The magnetic fields induced by the angular magnets in proximity of the nickel lines keep the magnetically labeled cells in fixed positions. This allows revisiting the detected events after measuring the fluorescence emissions and intensities for a more extensive analysis to further identify the detected events. One can microscopically view the images of such events and apply independent morphometric criteria to identify the events as actual cells.

The present invention provides a method and apparatus for analytical imaging of target entities, according to claims 1 and 12 respectively. Thus, the invention provides a novel scanning and imaging diagnostic system for detection, classification and enumeration of cells, which comprises an efficient automated means for collecting and aligning imunomagnetically labeled target cells from body fluids, and in which such collected cells also bear at least one immuno-specific fluorescent label that differentiates target from non-target cells labeled with different fluorescent label(s).

In accordance with the present invention, the new TDI scanning and imaging techniques can be integrated into a system such as the imaging system in US 10/612,144 to obtain high quality fluorescence images. The discoveries described and claimed herein have greatly improved the detection, enumeration and classification of rare cells over systems and methods in prior art. Efficient detection of cells at very low frequencies, so called rare events, requires minimal sample handling to avoid losses of cells. Furthermore, the volume from which the rare cells are separated and enriched should be as large as possible to increase the sensitivity of detection. With the development and application of the disclosed novel techniques, fluorescent images of specific events can now be obtained resulting in a highly accurate identification, thus making the inventive system a powerful tool for the detection of rare events in body fluids.

**Brief Description of the Drawings**

Figure 1

Object interpretation by TDI imaging. The object represented by the circle is moving along the vertical axis. When in the sensor area, the charge in the pixel well is coninuously increaseing with increasing exposure and is mvoing along with the passing cell. At time Ti+11 the end of the sensor is reached and the readout register reads the amount of chaarge integrated over all TDI stages.

Figure 2

a) Scatter plot of CD45 antibody-APC/Cy7 versus CAM5.2 antibody-APC fluorescence of SKBR3 cells spiked into whole blood, captured and aligned by EpCAM antibody-labeled magnetic nanoparticles. Some representative sub-images of the measured events of region 1, the SKBR3 cell region, and of the broad band containing the debris are shown. Region 2 is the region where the leukocytes would appear, if present, and aligned along the Ni lines.
b) Full image of an SKBR3 cell with its corresponding measured fluorescence signals.

Figure 3

Schematic representatio of a typical imaging system, disclosed in US 10/612,144.

Figure 4

A schematic representation of the image reconstruction method is shown. The detected events or cells are scanned with the laser by moving the stage that is equipped with an encoder. The CCD camera captures the individual sub-images and stores them in computer memory along with the corresponding encoder for positioning the x-y coordinates of the stage. After scanning is complete, the sub-images are combined to form a full reconstructed image of the object by using the encoder values, which are calculated back to the number of pixels that the subsequent sub-images should be shifted with respect to each other. Summation of the shifted sub-images gives the complete reconstructed fluorescent image of the cell.

Figure 5

a) Fluorescent signals captured when a homogeneous layer of dye is scanned.
b) Two graphs showing the sums of the fluorescent intensities in the x- and y-directions for the same scanned dye.

Figure 6

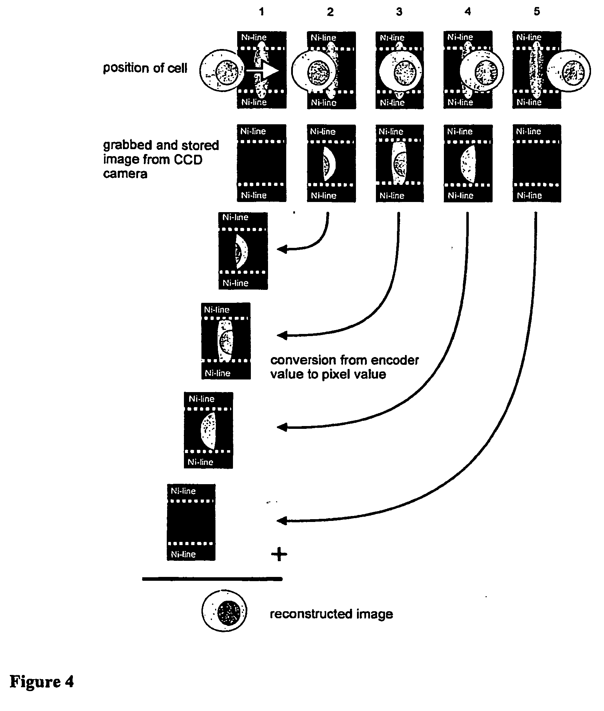

a) A graphical representation of the solid angle captured by the objective as a function of the position in the chamber. The numerical aperture (NA) of the compact disk (CD) objective in air is 0.45, resulting in an effective NA of 0.34 inside the chamber on the stage. The NA corresponds to a solid angle of 0.37 sr. The circle represents an aligned object with a diameter of 7 $\mu$m. The effective collecting angle of two points is indicated.
b) The graph shows the relative sum of the calculated solid angles of Figure 4a in the z-direction.

Figure 7

SNR measurements for several laser powers when scanning cells labelled with rhodamine 6G.

Figure 8

SNR measurements at different magnfications when scanning cells labelled with Rhodamine 6G and illuminated with a laser power of 10 mW.

Figure 9

Illustration of the CCD signal that is collecting charge from the dark current, readout noise and signal photons froma constant illumination and by a fluorescent dye. The vertical line is indicating the time when the SNR is optimal for the emission from a dye.
Figure 10.
Schematic representation of the Cell Tracks system with TDI. Exemplary Dual lasers (red/green) enable several types of dyes to be used.

Figure 11

Laser spot illuminating a sample. The nickel lines reflect light on the left and right sides.

Figure 12

Numbe of pixels that overlap due to misalignment.

Figure 13

Cone of light for illumination and collection.

Figure 14

Scatter plot of Oxazine 750 fluorescence versus CD4-APC fluorescence of white blood cells in whole blood captured and aligned by CD45-labeled magnetic nanoparticles. Some representative images of the monocyte and granulocyte regions are shown.

Figure 15

Time resolved imaging of Oxazine 750 stained CD45 ferrofluid captured leukocytes in whole blood utilizing the Cell Tracks system: time span is 0 to 120 sec at 20 sec intervals.

## DETAILED DESCRIPTION OF THE INVENTION

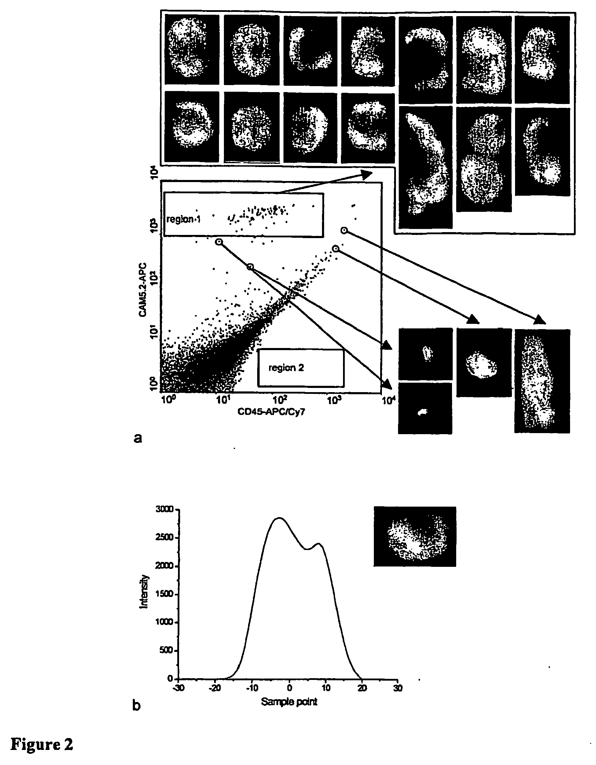

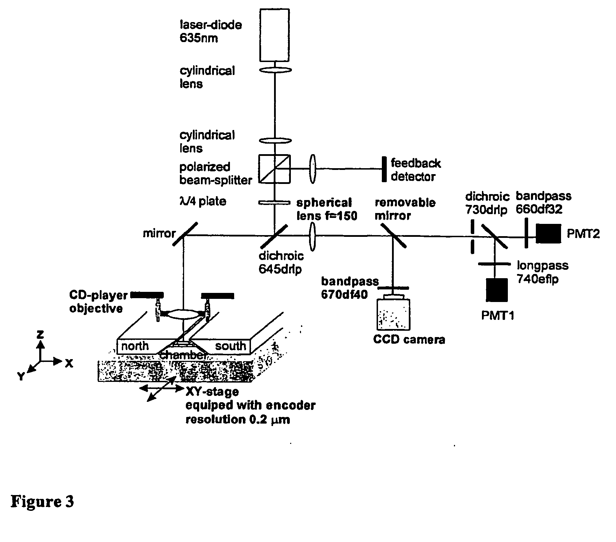

Racila et al. (Proc. Nat. Acad. Sci. 95, 4589-94,1998) described a method for separating breast carcinoma cells from blood using a sequence of steps including immunomagnetic labeling followed by immunophenotyping analysis in a flow cytometer. As stated in US 10/612,144, this imaging system is capable of immunophenotyping individual cells and confirming their idenity by providing a fluorescence image. The procedure was tested by the detection of cultured cells of the breast cancer cell line, SKBR3, spiked into whole blood. The spiked sample was prepared as described in the Examples. Figure 2a shows the scatter plot of the APC/Cy7 channel versus the APC channel in flow cytometry. The SKBR3 cells are located in Region 1. Debris appears as the broad band and leukocytes, if present, are located in Region 2. After measuring the scatter plot, some of the events were imaged with the novel imaging technique disclosed herein. After selecting an object or cell in the scatter plot, the imaging system automatically goes back to the measured position of the cell and the imaging routine is started. The set of images taken from events in Region 1 show cells with fluorescently stained cytoplasm. The nucleus of these cells is visible as a darker region. The images are different for debris, which is not located in Region 1. Figure 2b shows an image of an SKBR3 cell with its corresponding measured fluorescence signals. The fluorescent image and measured PMT signals correlate well.
As shown in Figure 3, the imaging system, disclosed in US 10/612,144, consists of a standard monochrome surveillance charge-coupled device (CCD) camera at a frame rate 25Hz, having manual gain adjustment (US 10/612,144). Using this arrangement with the insertion of a removable mirror into the light beam, the fluorescent light captured by the compact disk (CD) objective is focused by the spherical achromatic lens (f=150 mm) onto the CCD instead of a simple pinhole. The CD objective consists of a single aspherical lens, optimized to obtain a diffraction-limited spot size at a wavelength of 780 nm, similar to those used in current CD players. With a preferred numerical aperture (NA) of the lens is 0.45 and a lens diameter of 4 mm, the elliptical shape of the image is created by the two cylindrical lenses that are placed at a distance slightly larger than twice their individual focal lengths. The short axis of the elliptical focus is set at 4 $\mu$m (FWHM) which is smaller than the diameter of a cell, limiting the focus to one cell at any one time. The longer axis is larger than the Ni line spacing. The light focused on the Ni lines is reflected and used for feedback control. The Ni lines are present on a 0.5 mm thick glass substrate. Focusing the laser light (635nm) onto the Ni lines through the glass substrate with the CD-objective results in a non-homogeneous laser focus.

The imaging system is based on magnetic collection methods that does not stress or distort the cells as is commonly observed in cytospin systems using centrifugal deposition on slides. Hence, the magnetically aligned cells maintain their native three-dimensional shape and volume. The device and method described herein can also be used to obtain a confocal image of cells and, therefore, to enhance the image quality by allowing a more accurate determination of the 3D-distribution of the fluorescent dye inside the cells.

A full image of a measured event, wherein a full image is defined as a reconstructed image consisting of combined multiple digitized sub-images, is obtained by the acquistion of positional information. Before image acqisiton of a previously measured event, the event must be relocated on the sample chamber. Consequently, spatial information, such as x-y sample coordinates for each sub-image, is needed. To obtain positional information in the y-direction, the stage which moves both the magnets and the sample chamber has been equipped with an encoder that has a resolution of 0.2 $\mu$m. The line number for a sub-image of a specific event is measured to give positonal information in the x-direction. The encoder signals, together with the line number, are stored in memory and are coupled to the signals measured from the photomultiplier tube (PMT) for each sub-image. In this manner, all the measured sub-image events are associated with and indexed to an x-y position for the sample.

To return to the position where a specific event or sub-image was measured, the laser focus shifts by the number of lines equal to the current line number minus the line number on which the specific event was measured. Concurrently, the stage moves in the y-direction to the specfic encoder position. An alternative method for obtaining encoder signals is to add profiles to the Ni lines to record positon. This approach further permits the use of a significantly simpler and cheaper stage to move the sample. It should be further noted that while Ni lines are used to align the cells and to re-locate the cells using the line number on which the cell is measured with its corresponding encoder value the imaging invention described herein is not dependent on the presence of Ni lines or magnetic lines and can be used on any surface on which cells or other fluorescent objects of interest are present or can be deposited. Only the encoder data in the scan direction would be needed for reconstructing the image from the stored and grabbed sub-images.

The intensity profile at a particular position of the laser focus is non-homogeneous and, since the diameter of a cell is typically between 5 and 20 $\mu$m, it is also smaller than the cell diameter. Therefore, uniform illumination with a laser focus that is smaller than the cell diameter and has a non-homogeneous intensity profile is obtained by scanning the laser across the cell surface with the movement of an optical component in the beam, as is done in a laser scanning microscope (Corle, TR, Confocal Scanning Microscopy and Related Imaging Systems, Academic Press, NY, 1995). In the imaging system of US 10/612,144, uniform illumination by this method would result in a loss of feedback, which in turn would result in a loss of positional information in the x-direction. The intensity profile is obtained after adding the individual pixel intensities of the focal spot image in the y-direction. With a Ni line spacing of 10 $\mu$m, the summed intensity profile has an intensity variation of $\pm$6 % across the line spacing. Moving a cell in the y-direction through this focus has the result that every part of the cell has received an almost equal illumination after it has passed through the laser focus. This method is used to obtain a full high quality fluorescent image of an aligned cell, based on summation of individual sub-images.

The magnets and chamber are positioned on a stage that moves the cells through the focus in the y-direction. To obtain a full image of a specific cell, the laser focus is shifted to the line where the specific sub-image event was measured and the stage is moved in the y-direction to the corresponding encoder position with a speed of 10 mm/sec. The stage is slowed down to a speed of 5 um/sec when the distance to the cell position is 25 $\mu$m. While moving the stage at this low constant speed in the y-direction, the cell is scanned by the laser focus, and the fluorescence signal for each sub-image is captured on the CCD (Figure 4).

A frame grabber card captures the CCD sub-images at 25 Hz and these are stored in memory. In each subsequent sub-image, a different part of the cell is illuminated since the laser focus in the scan direction is smaller than the cell diameter. Together with the sub-image capture, the encoder position is read and both are stored in computer memory. A total of 40 $\mu$m are scanned, corresponding to 200 captured sub-images each with 150 x 250 pixels. There is an average of a 0.2 $\mu$m shift with respect to each other. This corresponds to a shift of 2.63 pixels on the CCD surface. Using the encoder values, the captured sub-images are shifted over a number of pixels corresponding to the difference in their associated encoder values x 2.63, which are then summed or combined. This results in a reconstructed full cell image as is illustrated in Figure 3. The sub-image resolution in the y-direction is determined by the encoder resolution (0.2 $\mu$m). The resolution in the x-direction is determined by the number of pixels in the image recorded in the x-direction (0.07 $\mu$m pixel). Both resolutions are smaller than the diffraction limit. Hence, it will be appreciated be those skilled in the art that the ultimate image resolution will not be determined by the encoder or the camera but by the imaging optics.

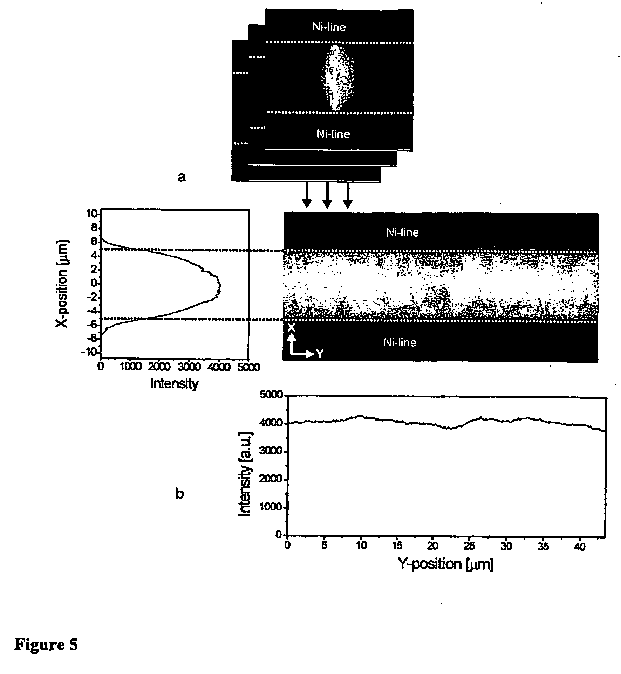

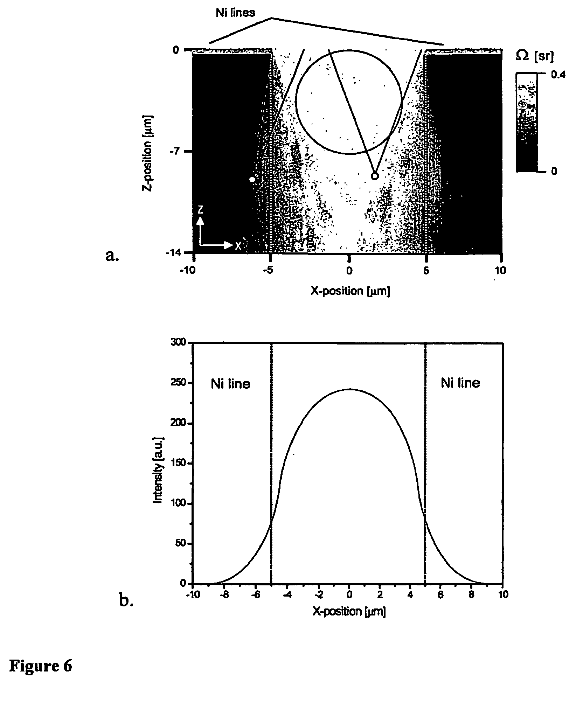

An integral component of the imaging optics of the present invention is the homogenity of illumination throughout the image. Thus, a profile of this homogenity was assessed with a thin layer of a concentrated fluorescent dye solution, placed in the same plane of the magnetically labeled cells such that they would be aligned between the Ni lines which are spaced 10 μm apart. The dye layer was scanned and imaged at a speed of 5 μm/sec as previously described. The sequentially captured sub-images are presented in Figure 5 a. For a uniform layer of fluorescent dye, one would expect a homogeneous fluorescent image, if the illumination were uniform. The obtained image is shown in Figure 5b. The observed signals, as measured in intensity units along the center trace in the y-direction of the image, were found to vary by ± 7 %. One explanation for this variation may be non-homogeneity of the dye layer, which would affect the emitted and captured fluorescent light. A second explanation is that the stage did not move with a constant speed. The position of the stage is not synchronized with the frame rate of the camera and the frame grabber card, but images were grabbed at 25 Hz regardless of the speed and position of the stage in the y-direction. If the stage moves faster than 5 μm/sec in a certain region, fewer images would be captured of this region resulting in a lower total intensity. However, the variation in the speed of the stage was measured and turned out to be much smaller than the observed variation in the measured image intensity. The apparent non-uniformity along the center trace must therefore be due the non-homogeneity of the dye layer. The intensity profile in the x-direction or perpendicular to the Ni lines and scan direction is also presented in Figure 5b. The intensity of the sub-images in this direction express a maximum centered between the Ni lines which falls off near the edges of the Ni lines. The Ni lines obstruct the emitted fluorescent light resulting in a smaller collecting angle, which in turn results in a smaller effective NA of the objective. In Figure 6a, the effective solid angle detected by the objective is calculated as a function of the position in the chamber. The simulation was performed using a line spacing of 10 μm and a depth or layer thickness of 14 μm. The graph in Figure 6b shows the sum of the calculated solid angle values of Figure 5a in the z-direction, thus providing a measure of the collected and measured intensities as a function of the x-position. The calculated intensity profile is in agreement with the measured intensities for the uniform dye layer. The effective NA of objects close to the Ni lines is largely reduced resulting in a non-uniform captured intensity in the x-direction even though uniform illumination is used. Objects with a diameter smaller than 7 μm, as is indicated by the circle in Figure 6a, are imaged without loss in intensity due to the shielding effect of the Ni lines, since the effective NA in this region is reduced by the Ni lines.

The preferred camera used in the present invention is a Dalsa eclipse high sensitivity TDI linescan camera. It is connected to a computer with a framegrabber board. The camera has 0 to 255 grey levels (8 bit) with a total of 512 horizontal pixels and 96 TDI stages in the vertical direction. The pixel size is 13 μm and the maximum line rate is 64 kHz.

Responsivity of a CCD is dependent upon the wavelength, expressed in Digital Numbers (DN). The Dalsa Eclipse line scanner has a high responsivity due to the high quantum efficiency in the visible region. This responsivity is typically 1950 DN/(nJ/cm$^2$) for a 0 dB gain in each column for the sensor. Responsivity also depends upon the pixel area, the Fill Factor of each pixel, the quantum efficiency of the CCD, QE, in electrons/photon, the number of TDI stages (96) and the electronic gain (dB). The fill factor of the camera is 100% which means that the entire area of the CCD sensor is photo-sensitive. The amount of photons that are actually converted to signal electrons depends on the quantum efficiency (QE) of the sensor. The QE efficiency is normally in the range of 0.4 to 0.7. While the photo-response for each pixel varies a bit, for the TDI CCD camera the photo-response uniformity is very good compared to a standard photodiode linear array. The photo response improves with the averaging over 96 TDI stages and the non-uniformity reduced by a factor of 1/96. With the same effect of averaging seen in the dark-current distribution, a good uniformity of the pixels results in the improved linear output of the camera.

Several noise sources are present in a CCD camera. The major sources are the readout noise, the photon noise, and dark current noise. These noises arise from the imaging array, the readout register, and the output structure. Photon noise is defined as photons incident on the CCD which are converted to photoelectrons within the silicon layer. These photoelectrons comprise the signal but also carry a statistical variation of fluctuations in the photon arrival rate at a given point. Readout noise is electronic noise which is inherent to the CCD. Readout noise refers to the uncertainty introduced during the process of quantifying the electronic signal on the CCD. Dark current noise is noise that arises from the statistical variation of thermally generated electrons. These electrons come from the CCD material through thermal vibration. All these noise sources contribute to the quality of the overall signal which is expressed as the signal to noise ration (SNR).

SNR describes the quality of a measurement. Specifically, it is the ratio of the measured signal to the overall measured noise at a pixel. High SNR is particularly important in applications requrieing low light measurement. Taken together, the SNR for each pixel for a CCD camera is dependent upon the following relation:

$$SNR = \frac{N_s \cdot \eta_q}{\sqrt{(N_s + N_b) \cdot \eta_q + I_d \cdot T_{int} + N_r^2}} \qquad (1)$$

where $T_{int}$ is the total integration time (seconds), $N_s$ is the number of signal photons hitting the CCD in a time $T_{int}$ (photons), $N_b$ is the number of background photons hitting the CCD in a time $T_{int}$ (photons), $I_d$ is the dark current (electrons/pixel/second), $N_r$ is the readout noise, and $n_q$ is the quantum efficiency.

In the imnging system without TDI, SNR is improved by reducing the imaging scan speed, resulting in larger number of captured sub-images of a specific event and a better signal to noise ratio. However, no improvement in image quality for the APC labeled SKBR3 cells is observed when the image scanning speed was reduced. The limit of the imaging scan velocity is determined by the photo-bleaching rate of the dye molecules. No fluorescence is detected with the CCD camera if an SKBR3 cell is scanned for the second time, indicating that most of the APC molecules have already been photo-destroyed after the first scan. Reducing the scan speed would, therefore, make no difference in the detected fluorescence signal and would only result in increased fluorescent background. The optimal scan speed is, therefore, dependent on the individual dye characteristics and is different for each fluorescent dye. Therefore, to the improve signal to noise ratio it may be better to scan faster. On the other hand scanning faster than 5 $\mu$m/sec when using a camera with a frame rate of 25 Hz would result in a loss of resolution since the captured images would be spaced more than 0.2 $\mu$m apart. A CCD camera with higher frame rate would be needed to scan faster without losing resolution. Replacing the standard surveillance CCD camera with a more sensitive camera would also allow imaging of dimly stained cells.

The SNR in an imaging system with a TDI camera as claimed in the present invention depends upon the camera, the total integration time and the amount of signal photons hitting the CCD during integration. These signal photons come from fluorescent dyes that are attached to the cells. The total amount of fluorescence signal photons is not constant for these dyes, and is compounded by a reduced total time due to bleaching of the dyes. The result is that at slow scanning speeds (long integration times) most of the fluorescence gathered results in a high SNR. However, the scanning speed can be too slow where the number of photons emitted per second will be so small that the dark current dominates the charge distribution which results in a lower SNR.

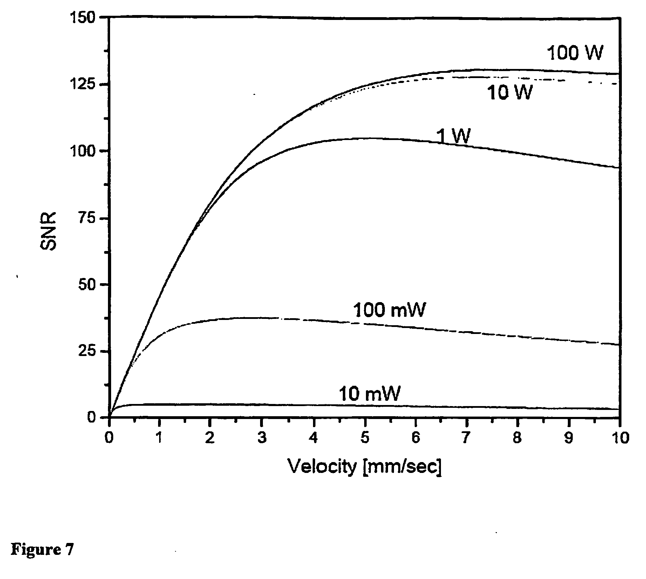

Consequently, the scanning speed has to be adjusted to the particular dye used to obtain the maximum SNR. A typical situation would be to determine the optimum scanning speed for the sample stage in the Y-direction when a fluorescence indicator is used in order to obtain the higest SNR with the TDI imager of the present invention. The dependence of the SNR will be on the bleaching rate, the laser illumination and magnification. Figure 7 shows the calculated SNR for rhodomine 6G with several laser powers. Measurements are based upon parameters in Table 1.

## Table 1.

| Simulation parameters | | |
|---|---|---|
| | | |
| Noise | | |
| Dark current ($N_d$) | 8000 | e-/pixel/sec |
| Readout noise ($N_r$) | 60 | e-/pixel |
| Back ground photons ($N_b$) | 0 | |
| | | |
| Illumination | | |
| Collection efficiency | 0.1 | |
| Aspect ratio | 0.3 | |
| Magnification | 65 | |
| | | |
| Cell/label | | |
| Dye | Rhodamine 6G | |
| # fluophores/cell | 10000 | # |
| Cell diameter | 10 | $\mu$m |

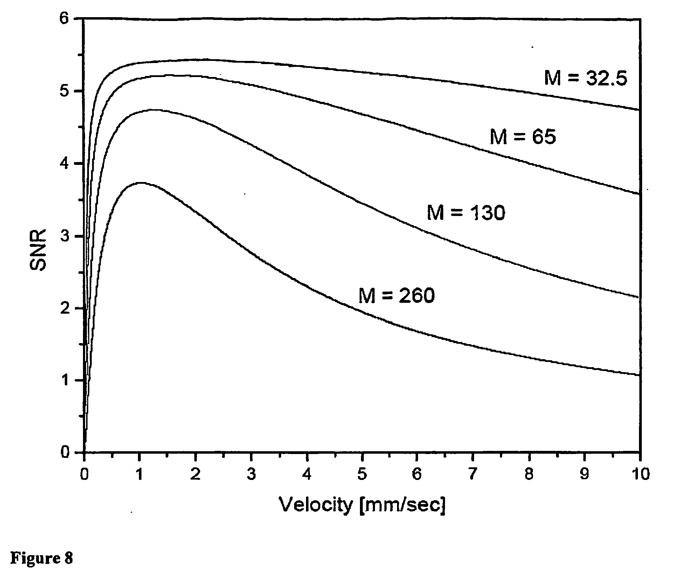

At a low scanning velocity, the SNR improves with increasing velocity. The SNR also improves with higher illumination power. Maximum SNR is obtained when the total amount of photons is increased to more than the amount of dark current photons. Accordingly when scanning too slowly, the SNR will decrease while the rate of photone emitted by the fluophores will not be high enough. When scanning to fast, smaller amounts of photons are collected, resulting in a decreased SNR. Figure 8 shows the calculated SNR for several magnifications for a laser power of 10 mW. The SNR improves when the magnification is lower with the density of photons on the CCD higher for a smaller object. Figure 7 shows an optimum speed of approximately 1.8 mm/sec for a 65X magnification and a 10 mW laser power. As the magnification is elevated (Figure 8), the optimum speed decreases.

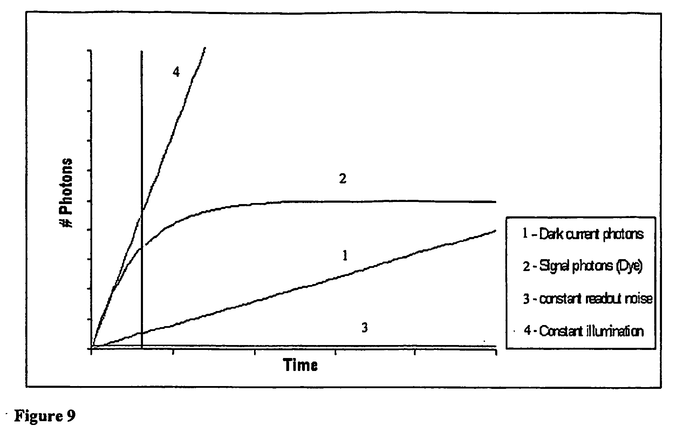

As mentioned above, SNR is also a function of the integration time. Figure 9 depicts the amont of photons that reach the CCD and the associated relation with SNR as a funtion of integration time. The optimum SNR for a constant illumination is constantly increasing and is limited by the amount of charge the pixel can hold. For the best performance, a high illumination rate will shorten the total collecitng time and reduce the total amount of dart current.

SNR for the imaging system with TDI would preferably scan the object with a velocity ranging from about 1 to 2.5 mm/sec. While not limiting the scope of the present invention, this SNR is a calculation based upon Rhodamine 6G, and depends upon the particular dye.

The original imaging system (US 10/612,144) used a red diode laser as an excitaiton source for several fluorescent labels. An encoder was used to give positional information of the cells and to relocate the aligned cells.

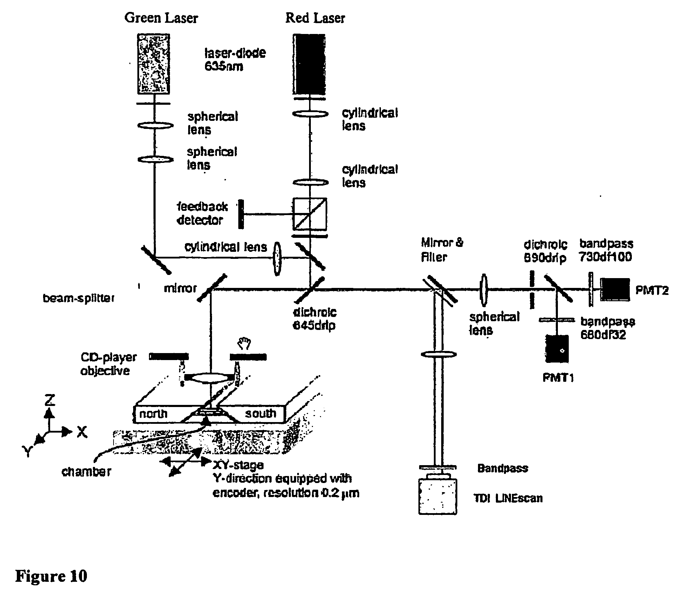

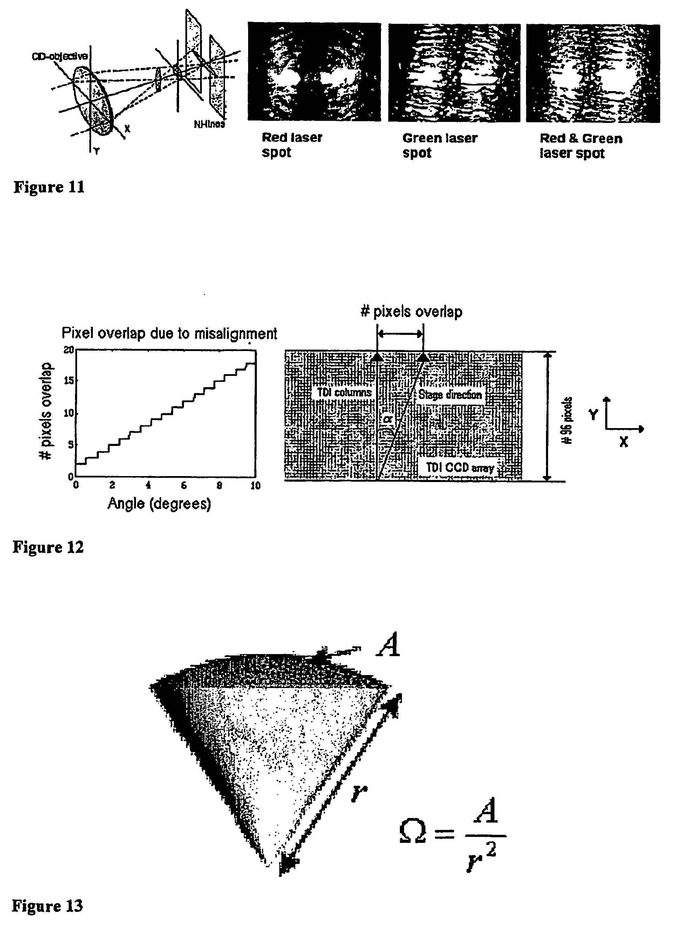

CellTracks with the TDI imager incorporates all red excitable dyes and the emissions of these dyes, along with a second green laser (532 nm) to extend the amount of dyes that can be used and to have an emission spectrum that is further apart (See Figure 10). This green laser beam is expanded 4 times with a beam expander. With a cylindrical lens, an elliptical spot is created which results in two focal planes after focussing with the CD objective (Figure 11). A dichronic mirror that reflects the green laser light and transmits the red laser light is used to combine the two laser lines. The green laser spot overlaps the spot of the red laser. An achromat replaces the CD lens to obtain a focal plane that is equal for the red and the green laser light in combination within the beam. This allows the beams to run in parallel. A mirror/dichroic combination is used to project the red fluorescence at a different position on the CCD which is followed by the green fluorescence. The fluorescent signal is then measured with the PMT or TDI camera as in the present invention. The green fluorescence is projected on the lafte area of the CCD and the fluorescence form the red laser excitation is projected on the right side of the CCD.

The magnification necessary for the image using a TDI camera depends on the resolution of the positional encode and the camera. The preferred encoder is a Heidenhein MT 2571 with a resolution of 0.2 $\mu$m. This encoder will give a TTL pulse every 0.2 $\mu$m, used to trigger the TDI camera and pulse results in one TDI row shift.

To achieve a square pixel image, the pixel size in the y-direction equals the size of a pixel in the x-direction. After each pulse the TDI row is moved with a coresponding distance lp = 13 $\mu$m on the CCD. This results in a magnification of 13/0.2 = 65 for this configuration. To use a different magnification, a counter is used. This counter gives 1 TTL pulse to the camera after 1,2,4,6,..... pulses of the encoder. With this counter, the following magnifications can be used:

$$M = \frac{13}{0.2 \cdot k}, k = 1,2,4,6... \qquad (2)$$

When the encoder is used, the set-up is constrained for a cerain magnification and it is essential that the magnification is correct to ensure that the movement of the object and the shifting of the TDI rows is in synchrony. A mismatch of the magnificiation of the stage and the camera will result in a bluring of the image. When the magnification is too large or too small the image will be stretched in the forward or reverse direction, respectively. When the magnification is in synchrony, the speed of the x-y stage will vary, but will be limited by the maximum and minimum line rate of the camera. The minimum line rate of the camera is approximately 100 Hz, corresponding to a line time of 0.01 seconds and a total exposure time of 0.96 seconds for all TDI stages. Also if no encoder is used, the line rate and speed of the x-y stage can have a fixed value for a certain magnification.

The encoder sends pulses to trigger the camera for control of the line rate. Also connected, the framegraber sends a pulse in LVDS format to the camera for each falling edge of the encoder signal. To verify the resolution of the encoder, the amount of steps is counted for a period of 5 msec. Counter values are measured with a sampling frequence of 20 KHz.

Image resolution, particularly laterally, depends on the wavelength, focal distance, and entrance diameter, all limited by the diffraction limit of the objective. The one objective considered in the present invention is a Sony CD pick-up system (KSS-210ARP). The accurator of this CD system is used with a focal distance of f = 3.8 mm, a numerical aperature ofN.A. = 0.45, and an opening entrance of D = 4 mm. The CD lens is designed to operate at a wavelength of 720 nm and, therefore, is expected to produce spherical and chromatic aberrations for other wavelengths which will reduce the resolution.

The resolution for the encoder is 0.2 $\mu$m for M = 65 and, with the shifting CCD rows, the illumination is always spread on two pixels, resulting in a resoluiton of 0.4 $\mu$m for the image. This resoluton is higher than the optical limit and is limited by the optical resolution. In the X-direction, a mismatich between the scanning direction and the TDI column results in a decrease of resolution. The movement of the cell should be parallel with the TDI columns. The number of overlaping pixels increases with a higher deviation angle (Figure 12). In the Y-direction, a mismatch of the magnification will also cause a reduced resolution with a blurring effect due to overlapping pixels.

The collection and illumination efficiency is determined by the light gathering power (N.A.) of the lens. In the present invention, the CD-lens is replaced with the achromatic lens in the pickup accurator. The achromatic lens has a focal distance of 7.5 mm and an entrance opening of 6.5 mm. The N.A. is approximately 0.27. The solid angle (Figure 13) is 0.21 with the achromatic lens, replacing the solid angle of 0.72 for the CD lens. The achromat is also used for the green laser inorder to have the same folcal distance. However, the solid angle of the achromat is much smaller than the solid angle in the CD lens. The solid angle is proportional to the intensity of the collected light, making the CD lens more efficient than the achromat. Although the achromatic lens is limiting the sensitivity (by approximately a factor of 15 for both illumination and collection of photons), it is more convenient to implement with a second laser, making tracking and focusing of the system possible. An achromat with a higher N.A. would be preferable, with a numerical aperature (N.A. > 0.5). This results in a higher SNR and a higher resolution. The present invention is also considered with the use of a microscope objective, although this type of objective would mandate changing the tracking and focusing mechanism.

For imaging and spectral separation of the fluorescent emission, a prism or combination of filters can be used. Instead of using a dichroic mirror combination, a prism is inserted in front of the CCD. With a direct vision prism, the deflection angle depends on the wavelength and the deflection angle is almost parallel with the incomign ray of light. To obtain an image where the fluorescence is separated with no spectral overlap, the spectral separation must be large enough to separate more than the maximum cell size. For example, with a maximum cell size of 20 $\mu$m the emission of APC and PE is about 100 nm apart and the bandwidth of emission is approximately 10 nm, resulting in a pixel resolution of 2 $\mu$m in the X-direction. Thus, dyes with smaller emission spectrums are required for optical resolutions of 0.7 to around 1 $\mu$m. A dichroic/mirror combination can also be used whereby a high pass filter and an almost parallel mirror can be used to separate the fluorescent emission into two disticnt areas. For example, the emission of PE excited with the green laser is reflected on the filter surface while the emission of APC is transmitted by the filter and reflected on the mirror.

Implementation of TDI imaging into the imaging systems like CellTracks provides a method with good image resolution and an improved signal to noise compared to the analysis with the scanning mirror and the full frame CCD. The scan speed is also increased with the time to image one line under TDI imaging around 10 sec (at a speed of 1.5 mm/sec). Previous methods would only measure 1 cell within this time period. With the dual excitation laser, it is possible to image the fluorescent emission with more than one dye. Dyes that have the same absorption spectrums but different emission spectrums have an emission that is close together, demanding a narow bandwidth of the filters to image the emission of these dyes.

It will be apparent to those skilled in the art that the improved scanning and imaging system of the invention is not to be limited by the foregoing descriptions of preferred embodiments, and that the preferred embodiments of the invention which incorporate these improvements, as previously described, have also been found to enable the invention to be employed in many fields and applications to diagnosis of cells and to particulate target species in general. The following Examples illustrate specific of the invention, but are not thereby limited in scope.

Example 1

For these experiments, 10 $\mu$l of fixed SKBR3 cells (50,000 cells/ml) were mixed with 290 $\mu$l of EDTA blood. Also added at the same time were 100 $\mu$l of magnetic ferrofluid coated with anti-EpCAM (magnetic particles of about 200 nm size coated with proteins, streptavidin and biotinylated EpCAM antibody), an antibody specific for epithelial cells and known to be present on SKBR3 cells (cultured at Immunicon Corp., Huntingdon Valley, PA), 10 $\mu$l of allophycocyanin (APC) conjugated to monoclonal antibodies recognizing anti-cytokeratin species or cytoskeletal proteins present in epithelial cells (e.g. SKBR3 cells that are epithelium derived) and 10 $\mu$l CD45-APC/Cy7 (Caltag, Burlingame, CA) to identify leukocytes and identify leukocytes that may nonspecifically bind to cytokeratin antibody. After 15 minutes incubation, 50 $\mu$l of this blood reaction mixture was injected into the chamber. The chamber was placed in the Cell Tracks magnet assembly and after two minutes collection time, the feedback system was switched on and the measurements were started. In a single measurement, 40 lines with aligned cells, each 15 mm in length and with a line period of 30 $\mu$m were scanned. At a chamber height of 0.5 mm, the scanned volume represents 9 $\mu$l. The results of scanning the collected labeled SKBR3 cells with the corresponding measured immuno-fluorescent signals are shown in Figures 2a and 2b. Figure 2a shows a scatter plot of CD45-APC /Cy7 dye versus CAM5.2 antibody-APC fluorescence of SKBR3 cells in whole blood, captured and aligned by EpCAM-labeled magnetic nanoparticles. Some representative images of the measured events of Region 1, the SKBR3 cell region, and of the broad band containing the debris are shown. Region 2 is the region where the leukocytes would appear, if present and aligned along the Ni lines. Figure 2b shows an image of one SKBR3 cell with its corresponding measured fluorescence signals.

Example 2

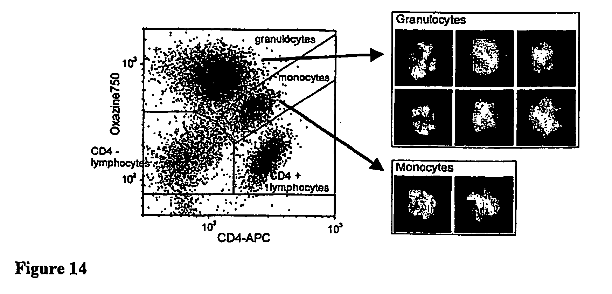

In this experiment 100 $\mu$l of EDTA anti-coagulated blood, 50 $\mu$l of ferrofluid containing 5$\mu$g of CD45-labeled ferromagnetic nanoparticles, 1.5 $\mu$l CD4-APC and 25 $\mu$l of $10^{-5}$ M Oxazine 750 perchlorate (Exciton Inc., Dayton, OH) were added. The optimum concentration of the reagents was obtained by serial titration of each of the reagents. After incubation for 15 minutes, 300 $\mu$l PBS was added and 50 $\mu$l of the blood mixture was placed into the capillary that was already placed between the magnets. The capillary has a glass bottom shaped in a way that it fits between the 70° tilted faces of the magnets. Two strips of double-sided tape with a thickness of 0.5 mm (3M Co., St. Paul, MN) were placed on the glass with spacing of 3mm to form the sidewalls of the capillary. Ni lines, about 30$\mu$m wide and about 0.2$\mu$m thick, were produced by standard photolithographic techniques on a 7740 Pyrex® glass wafer (Corning International, Germany). Wafers were cut in pieces of 4 mm x 25 mm and these were placed, with the Ni lines facing the bottom, on the double sided tape to form the top of the capillary. The inner dimensions of the capillary are height= 0.5 mm, length = 25 mm, width = 3 mm. In the measurement presented here the scan speed in the y-direction was 4 mm/sec, the chamber was scanned over 15 mm and 40 lines were scanned, resulting in a measuring time of two and a half minutes. Since the period of the lines is 30 $\mu$m, the surface scanned is 18 mm$^2$. As the height of the chamber was 0.5 mm, the scanned volume is 9 $\mu$l. For the differential white blood cell count, the addition of reagents resulted in a dilution factor of 4.77. To shorten the time that the cells need to align between lines and to assure that even the weakly magnetic labeled cells would be attracted to the upper surface, the capillary together with the magnets was placed upside down after the blood was placed into the capillary. After two minutes the capillary with the magnets was inverted again and, after approximately one minute, the feedback system was switched on and the measurement was started. To separate the emission spectra, a 660df32 band-pass filter for the APC fluorescence and a 730df100 band-pass filter (both filters from Omega Optical Co., Brattleboro, VT) for the Oxazine 750 were used. As the fluorescence intensity of Oxazine 750 stained cells is significantly greater than that of immuno-fluorescent CD4-APC labeled cells, compensation of the spectral overlap is required. A typical example of the scatter plot obtained after compensation is shown in Figure 14. Four populations are clearly visible and were identified as CD4+ lymphocytes, CD4+ monocytes, CD4- lymphocytes and neutrophilic granulocytes. The gate settings illustrated in the figure were used to determine the number of events in each gate. Total number of leukocytes measured was 12,350 and the measuring time was 2.5 minutes. To examine the distribution of the fluorescence from the detected objects, software was written to allow the user to point at the object of interest in the scatter plot. The system then moved to the location of this event and an image was taken. Surprisingly, the images clearly demonstrated that the fluorescence obtained from the Oxazine 750 staining was not derived from the nucleus but from the granules (Shapiro HM, Stephens S: Flow cytometry of DNA Content Using Oxazine 750 or Related Laser Dyes With 633 nm Excitation. Cytometry 1986; 7: 107-110). Six images obtained from the events in the granulocyte gate and two images from events in the monocyte gate are shown.

Example 3

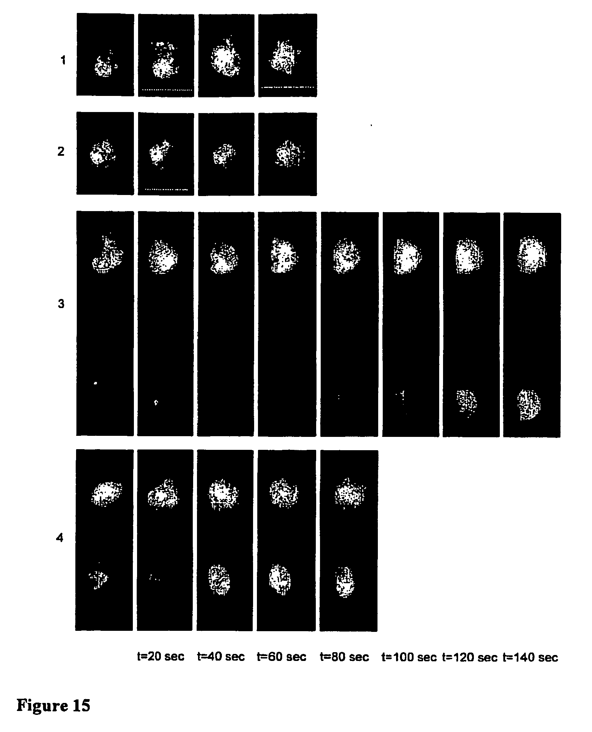

The experiment described in example 2 was repeated but time resolved images were taken with the CellTracks imaging system from Oxazine 750 stained and CD45 ferrofluid captured leukocytes in whole blood. Figure 15 shows four examples of images taken at 20 seconds intervals. The distribution of the fluorescence within the cells is clearly changing between the time intervals and different cells behave differently as is obvious from the cells followed in frame 3 and 4. In both frames images from two cells in close proximity are taken and the differences in uptake and cellular distribution of the Oxazine 750 are apparent From these examples it is obvious that this imaging system has a unique capability to perform functional analysis of cells as, for example, one can study the responses of cells in blood to drugs or other components in real time.

Example 4

TDI incorporation in the CellTracks imaging system was tested with fluorescent beads and HELA tumor cells. A non-cooled Dalsa Eclipse camera with a 96 CCD surface was used. The stage, moving with the sample chamber, was equipped with an encoder that has a resolution of 0.2 $\mu$m. The TDI rows shifted on every encoder pulse which guaranteed a one to one relationship between a moving cell and its image onto the CCD. HELA tumor cells were labeled with anti-cytokeratine-PE and the nucleus stained with DAPI. A brightfield image was made with 880 nm IR-LED. TDI imaging resulted in rapid imaging of cells with good SNR at an optimum scanning speed.

**Claims**

1.   A method for analytical imaging of target entities, which method comprises:

  a. obtaining a sample suspected of containing said target entities,
  b. magnetically labeling said target entities with magnetic particles that are specific for said target entities,
  c. magnetically manipulating said target entities towards a collection surface in a sample chamber,
  d. moving said sample chamber on a scanning stage while illuminating through an achromatic lens said collected target entities,
  e. detecting emitted light from said collected target entities using TDI (Time Delay Integration), and
  f. assessing characteristics of collected target entities from a group consisting of intensity, color, morphology, and combinations thereof.

2.   The method of Claim 1, further comprising:

  a. detecting emitted light whereby sequential sub-images of said collected target entities are collected, and
  b. re-combining said sub-images to construct a complete image of said collected target entities.

3.   The method of Claim 1, in which said illumination step further comprises the use of multiple wavelength light sources

4.   The method of Claim 1, in which said illuminating is by excitation with a laser.

5.   The method of Claim 3, in which said excitation is with a blue and green laser.

6.   The method of Claim 1, in which said detecting is by fluorescent emission.

7.   The method of Claim 1 wherein said emitted light is from a group consisiting of green fluorescent emitted light, blue fluorescent emitted light, brightfield light, and combinations thereof.

8. The method of Claim 1, in which said target entities are from a group consisting of epithelial cells, endothelial cells, tumor cells, cell debris, and combinations thereof.

9. The method of Claim 1, in which said magnetic labels are colloidal magnetic particles specific for an antigenic site on the target entity.

10. The method of Claim 9, in which said colloidal magnetic particles are specific for the Epithelial Cell Adhesion Molecule (EpCAM).

11. The method of Claim 1, in which said collection surface comprises parallel Nickel lines on a glass substrate.

12. An improved apparatus for analytical imaging of target entities having a sample chamber with a collection surface, an arragnement of magnets cabable of manipulating magnetically labeled target entities towards said collection surface, at least one light source, and a computer, whereby said apparatus comprises:

   a. a scanning stage with said sample chamber affixed for controlled speed and positioning, and is **characterised by**
   b. a TDI camera;
   c. an achromatic lens for illuminating said target entities.

13. The apparatus of Claim 12, in which said collection surface comprises Nickel lines on a glass substrate.

14. The apparatus of Claim 12, in which said light source is a laser.

**Patentansprüche**

1. Verfahren zum analytischen Abbilden von Zieleinheiten, wobei das Verfahren umfasst:

   a) das Erlangen einer Probe, welche vermutlich die Zieleinheiten enthält,
   b) das magnetische Kennzeichnen der Zieleinheiten mit magnetischen Partikeln, die spezifisch sind für die Zieleinheiten,
   c) das magnetische Beeinflussen der Zieleinheiten in Richtung auf eine Sammelfläche in einer Probenkammer,
   d) das Bewegen der Probenkammer auf einem Scantisch, während die gesammelten Zieleinheiten durch eine achromatische Linse beleuchtet werden,
   e) das Detektieren von emittiertem Licht von den gesammelten Zieleinheiten unter Verwendung von TDI (Time Delay Integration) und
   f) das Beurteilen von Eigenschaften von gesammelten Zieleinheiten aus einer Gruppe bestehend aus Intensität, Farbe, Morphologie und Kombinationen daraus.

2. Verfahren nach Anspruch 1, darüber hinaus umfassend:

   a) das Detektieren von emittiertem Licht, wobei sequentielle Teilabbildungen der gesammelten Zieleinheiten gesammelt werden, und
   b) das Rekombinieren der Teilabbildungen zur Erstellung einer vollständigen Abbildung der gesammelten Zieleinheiten.

3. Verfahren nach Anspruch 1, wobei der Beleuchtungsschritt darüber hinaus die Verwendung von Lichtquellen mit mehreren Wellenlängen umfasst.

4. Verfahren nach Anspruch 1, wobei die Beleuchtung durch Anregung mit einem Laser erfolgt.

5. Verfahren nach Anspruch 3, wobei die Anregung mit einem blauen und grünen Laser erfolgt.

6. Verfahren nach Anspruch 1, wobei das Detektieren mittels Fluoreszenzemission erfolgt.

7. Verfahren nach Anspruch 1, wobei das emittierte Licht aus einer Gruppe bestehend aus grün fluoreszierendem emittierten Licht, blau fluoreszierendem emittierten Licht, Hellfeldlicht und Kombinationen daraus ist.

**EP 1 656 545 B1**

8. Verfahren nach Anspruch 1, wobei die Zieleinheiten aus einer Gruppe bestehend aus Epithelzellen, Endothelzellen, Tumorzellen, Zelltrümmern und Kombinationen daraus sind.

9. Verfahren nach Anspruch 1, wobei die magnetischen Kennzeichnungen kolloidale magnetische Partikel sind, welche spezifisch für eine antigene Lokalisation auf der Zieleinheit sind.

10. Verfahren nach Anspruch 9, wobei die kolloidalen magnetischen Partikel spezifisch für das Epithelzellen-Adhäsions-Molekül (EpCAM) sind.

11. Verfahren nach Anspruch 1, wobei die Sammelfläche parallele Nickellinien auf einem Glassubstrat umfasst.

12. Verbesserte Vorrichtung zum analytischen Abbilden von Zieleinheiten mit einer Probenkammer mit einer Sammelfläche, einer Anordnung von Magneten, die in der Lage sind, magnetisch gekennzeichnete Zieleinheiten in Richtung auf die Sammelfläche hin zu beeinflussen, wenigstens einer Lichtquelle und einer Datenverarbeitungsanlage, wobei die Vorrichtung umfasst:

   a) einen Scantisch mit der Probenkammer, fixiert für kontrollierte Geschwindigkeit und Positionierung, und wobei sie **gekennzeichnet ist durch**
   b) eine TDI-Kamera;
   c) eine achromatische Linse zur Beleuchtung der Zieleinheiten.

13. Vorrichtung nach Anspruch 12, wobei die Sammelfläche Nickellinien auf einem Glassubstrat umfasst.

14. Vorrichtung nach Anspruch 12, wobei die Lichtquelle ein Laser ist.


**Revendications**

1. Procédé d'imagerie analytique d'entités cibles, ledit procédé comprenant :

   a. l'obtention d'un échantillon suspecté de contenir lesdites entités cibles,
   b. l'étiquetage magnétique desdites entités cibles avec des particules magnétiques spécifiques pour lesdites entités cibles,
   c. la manipulation magnétique desdites entités cibles vers une surface de collecte dans une chambre à échantillon,
   d. le déplacement de ladite chambre à échantillon sur un étage de balayage exploratoire, pendant l'illumination, par l'intermédiaire d'une lentille achromatique, desdites entités cibles collectées,
   e. la détection de la lumière émise émanant desdites entités cibles collectées, par utilisation d'une TDI (Intégration Temporelle - Time Delay Integration), et
   f. l'évaluation des caractéristiques des entités cibles collectées d'après un groupe composé de l'intensité, la couleur, la morphologie et des combinaisons de celles-ci.

2. Procédé selon la revendication 1, comprenant en outre :

   a. la détection de la lumière émise, de manière que des sous-images séquentielles desdites entités cibles collectées soient collectées, et
   b. recombinaison desdites sous-images, pour construire une image complète desdites entités cibles collectées.

3. Procédé selon la revendication 1, dans lequel ladite étape d'illumination comprend en outre l'utilisation de sources de lumière à longueurs d'ondes multiples.

4. Procédé selon la revendication 1, dans lequel ladite illumination est effectuée par excitation avec un laser.

5. Procédé selon la revendication 3, dans lequel ladite excitation est effectuée avec un laser bleu et un laser vert.

6. Procédé selon la revendication 1, dans lequel ladite détection est effectuée par émission de fluorescence.

7. Procédé selon la revendication 1, dans lequel ladite lumière émise est choisie dans un groupe composé de lumière émise à fluorescence verte, lumière émise à fluorescence bleue, lumière fond clair, et des combinaisons de celles-ci.

8. Procédé selon la revendication 1, dans lequel lesdites entités cibles sont choisies dans un groupe composé de cellules épithéliales, cellules endothéliales, cellules tumorales, débris cellulaires et des combinaisons de ceux-ci.

9. Procédé selon la revendication 1, dans lequel lesdites étiquettes magnétiques sont des particules magnétiques colloïdales, spécifiques à un site antigénique sur l'entité cible.

10. Procédé selon la revendication 9, dans lequel lesdites particules magnétiques colloïdales sont spécifiques à la Molécule d'Adhérence Cellulaire Epithéliale (EpCAM).

11. Procédé selon la revendication 1, dans lequel ladite surface de collecte comprend des lignes en Nickel sur un substrat en verre.

12. Dispositif amélioré d'imagerie analytique d'entités cibles comprenant une chambre à échantillon avec une surface de collecte, un agencement d'aimants capable de manipuler magnétiquement des entités cibles étiquetées vers ladite surface de collecte, au moins une source de lumière, et un ordinateur, ledit appareil comprenant :

   a. un étage de balayage exploratoire, ladite chambre à échantillon étant fixée pour obtenir une vitesse et un positionnement contrôlés, et **caractérisé par**
   b. une caméra TDI (Time Delay Intégration) ;
   c. une lentille achromatique, pour illuminer lesdites entités cibles.

13. Dispositif selon la revendication 12, dans lequel ladite surface de collecte comprend des lignes en Nickel sur un substrat en verre.

14. Dispositif selon la revendication 12, dans lequel ladite source de lumière est un laser.

**Figure 1**

a

b

**Figure 2**

**Figure 3**

EP 1 656 545 B1

Figure 4

19

Figure 5

a.

b.

**Figure 6**

**Figure 7**

**Figure 8**

**Figure 9**

**Figure 10**

**Figure 11**

**Figure 12**

$$\Omega = \frac{A}{r^2}$$

**Figure 13**

**Figure 14**

27

t=20 sec   t=40 sec   t=60 sec   t=80 sec   t=100 sec  t=120 sec  t=140 sec

**Figure 15**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6151405 A, Douglass
- US 5432054 A, Saunders
- US 10612144 B

- WO 02054339 A1
- WO 03014400 A1
- US 20020012910 A1

**Non-patent literature cited in the description**

- **RACILA et al.** *Proc. Nat. Acad. Sci.,* 1998, vol. 95, 4589-4594
- **TIBBE et al.** *Nature Biotech.,* 1999, vol. 17, 1210-13
- **RACILA et al.** *Proc. Nat. Acad. Sci.,* 1998, vol. 95, 4589-94

- **CORLE, TR.** Confocal Scanning Microscopy and Related Imaging Systems. Academic Press, 1995
- **SHAPIRO HM ; STEPHENS S.** Flow cytometry of DNA Content Using Oxazine 750 or Related Laser Dyes With 633 nm Excitation. *Cytometry,* 1986, vol. 7, 107-110